Europäisches Patentamt

European Patent Office

Office européen des brevets

(19)

(11) Veröffentlichungsnummer : **0 392 300 B1**

(12) **EUROPÄISCHE PATENTSCHRIFT**

(45) Veröffentlichungstag der Patentschrift :
**26.08.92 Patentblatt 92/35**

(51) Int. Cl.⁵ : **A61K 37/66, A61K 45/06**

(21) Anmeldenummer : **90106221.6**

(22) Anmeldetag : **31.03.90**

(54) **Verwendung von mindestens ein Cytokin zur Herstellung eines Arzneimittels zur systemischen Behandlung von präneoplastischen Läsionen.**

(30) Priorität : **11.04.89 DE 3911720**
**21.02.90 DE 4005416**

(43) Veröffentlichungstag der Anmeldung :
**17.10.90 Patentblatt 90/42**

(45) Bekanntmachung des Hinweises auf die
Patenterteilung :
**26.08.92 Patentblatt 92/35**

(84) Benannte Vertragsstaaten :
**AT BE CH DE DK ES FR GB GR IT LI LU NL SE**

(56) Entgegenhaltungen :
EP-A- 0 077 063
EP-A- 0 170 843
EP-A- 0 233 629
WO-A-83/01198
US-A- 4 605 555

(56) Entgegenhaltungen :
JOURNAL OF BIOLOGICAL RESPONSE MODI-
FIERS. vol. 7, no. 4, 1988, NEW YORK US
Seiten 384 - 389; BREGMAN M.D. et al:
"Humanrecombinant alpha- and gamma-interferons enhance the cytotoxic properties of
tumor necrosis factor on human melanoma."
JOURNAL OF BIOLOGICAL RESPONSE MODI-
FIERS. vol. 6, no. 6, 1987, NEW YORK US
Seiten 599 - 609; TAGUCHI T. et al:
"Augmentationof cytotoxic activity of recombinant human tumor necrosis factor by recombinant human interferon-gamma."
Biological abstract no 75027547 RANSOM J.H.
et al:"Lymphotoxin enhances the susceptibility of neoplastic and preneoplasticcells to
natural killer cell mediated destruction" &
INT.J.CANCER 29(4),1982,451-458.

(73) Patentinhaber : BOEHRINGER INGELHEIM
INTERNATIONAL GmbH
Postfach 20
W-6507 Ingelheim am Rhein (DE)

(72) Erfinder : Boniver, Jacques, Prof.
Rue Florikosse 28c
B-4802 Heusy-Verviers (BE)

EP 0 392 300 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Gegenstand der vorliegenden Erfindung ist die Verwendung von Arzneimitteln zur systemischen, therapeutischen und prophylaktischen Behandlung von präneoplastischen Läsionen, welche als Wirkstoff mindestens ein Cytokin enthalten, vorzugsweise mindestens ein Interferon (IFN) oder ein Tumornekrosefaktor (TNF) oder eine Kombination von diesen beiden Cytokinen, beispielsweise IFN-gamma (IFNγ) oder TNF alpha (TNFα) oder eine Kombination davon.

Insbesondere richtet sich die erfindungsgemäße Verwendung dieser Arzneimittel gegen solche präneoplastischen Läsionen, die nicht oder noch nicht klinisch oder morphologisch in Erscheinung getreten sind und aufgrund einer Exposition oder Behandlung durch physikalische und/oder chemische Mittel bei Säugetieren, besonders beim Menschen, kausal einhergehen und zu malignen Entartungen führen können.

Soweit bekannt, wurde die systemische Verwendung der Cytokine bisher nur zur Behandlung von manifesten Krebs-bzw. Tumorerkrankungen, d.h. nach erfolgter neoplastischer Transformation von bestimmten Zell- bzw. Gewebetypen, eingesetzt und in einigen Fällen eine Wirkung in vitro und in vivo nachgewiesen (Berry, S.F. et al., J. Immunol. 135, 1165-1171, 1985; Ruddle N.H., Immunol. Today 8, 129, 1987; Bentler B. & Cerami, A., New Engl. J. Medicine 316, 379-382, 1987).

Bekannt ist auch die lokale oder topische Verwendung von Interferon enthaltenden Gelen zur Behandlung bestehender präkanzeröser Veränderungen äußerer Gewebebereiche, wie z.B. Haut oder Mukosa (WO 83/01198). Eine systemische Behandlung wird als nicht wirksam oder, wegen einer möglichen zu hohen Dosierung, als nachteilig oder schädlich beurteilt. Die von der WO 83/01198 vermittelte Lehre wird von der US 4 605 555 aufgegriffen und offenbart gleichsam die ausschließlich externe, d.h. topisch oder lokale Verabreichung von Interferon enthaltenen Gelen, Salben, Pasten, Flüssigkeiten oder Sprays.

Die Europäische Patentanmeldung EP-A 0170843 offenbart eine synergistische Mischung bestehend aus TNF und Interferonen des Typs I und II zur Behandlung neoplastischer Erkrankungen.

Von Bregman, M.D. et al., J. Biol. Resp. Mod. 7, 384-389, 1988, wird die Wirksamkeit von TNF und einer Kombination von TNF mit IFNα oder IFNγ bei menschlichen Melanoma-Zellinien beschrieben, wonach sich eine Kombination von TNF mit IFNγ als am wirksamsten herausstellte.

Zu ähnlichen Ergebnissen kommt die Publikation von Taguchi, T. et al., J. Biol. Resp. Mod. 6, 599-609, 1987, wonach für eine Mischung aus humanem TNF mit humanem IFNγ ein stärkerer Antitumor-Effekt in vitro nachgewiesen wurde als mit TNF allein.

Da die bisherige Bekämpfung von bestehenden Krebsleiden selbst durch die moderneren Methoden nicht die erhofften Erfolge zeigte, muß der Behandlung von präneoplastischen Läsionen, insbesondere der Verhinderung oder Unterbrechung des Prozesses zur neoplastischen Transformation, eine besondere Bedeutung eingeräumt werden.

Der vorliegenden Erfindung lag demnach die Aufgabe zugrunde, Mittel bereitzustellen, die eine systemische therapeutische und prophylaktische Behandlung von präneoplastischen Läsionen ermöglichen, mit dem Ziel, den Prozeß zur Transformation zum malignen Tumor zu verhindern oder zu unterbrechen.

Zur Lösung dieser Aufgabe wurde überraschenderweise gefunden, daß die systemische Verwendung von Cytokinen, vorzugsweise IFN oder TNF oder eine Kombination davon, beispielsweise Interferon-gamma (IFNγ) oder Tumornekrosefaktor-alpha (TNFα) oder eine Kombination davon, die Progression von präneoplastischen Zellen, vorzugsweise von solchen, die aufgrund physikalischer oder chemischer Einwirkungen induziert werden, in Richtung Transformation zur malignen Entartung verhindert.

Es wurde unerwartet gefunden, daß wiederholte Injektionen von IFN, beispielsweise IFNγ, oder TNF, beispielsweise TNFα oder Kombinationen davon, die Entwicklung von Thymuslymphomen bei Mäusen verhindert, die vorher einer leukemogenischen Bestrahlung nach bekannter Methode (Kaplan H.S., Cancer Res. 27, 1325-1340, 1967, Defresne, M.P. et al., J. Nat. Cancer Inst. 77, 1079-1085, 1986) ausgesetzt worden waren. Dies zeigt eindeutig, daß diese Cytokine über die systemische Route, neben ihrer bekannten Antitumorwirkungen in vitro und in vivo, auch auf präneoplastische Gewebe wirken und den weiteren, in mehreren Schritten ablaufenden Prozess zur malignen Entartung unterbrechen, der sonst zur Entwicklung von Lymphomen führen würde. Ein ähnlicher Effekt auf präneoplastische Zellen wurde soweit bekannt bisher nicht beschrieben.

Demgemäß wäre zu postulieren, daß IFN und/oder TNF die präneoplastischen Zellen in ihren ursprünglichen Zustand zurückbilden können.

Beispielhaft wurde dieser Effekt dadurch belegt, daß durch die systemische Verwendung von IFNγ und/oder TNFα eine signifikante Reduktion der Mortalität und des Auftretens von strahleninduzierten Thymuslymphomen erzielt werden konnten.

Die erfindungsgemäß zu verwendenden Cytokine umfassen daher alle präneoplastischen Läsionen, die insbesondere aufgrund physikalischer und/oder chemischer Einwirkung auf einen Säugetierkörper entstehen können und die mit einiger Wahrscheinlichkeit in ein malignes Krebsstadium übergehen. Zu den präneoplastischen Läsionen, die erfindungsgemäß zu behandeln sind, zählen demnach insbesondere solche, die besonders bei Patienten entstehen, wenn diese chemische und/oder physikalische Behandlungen erhalten und somit einem erheblichen Risiko ausgesetzt sind, maligne Tumoren zu entwickeln. Bei derartigen Patienten besteht insbesondere das Risiko, myelodysplasisches Syndrom oder akute myeloische Leukämie, die nach einer Ganzkörperbestrahlungen, nach Exposition mit Benzol oder nach einer Behandlung mit alkylierenden Agentien zur Behandlung der Hodgin-Krankheit (10% dieser Patienten besitzen das Risiko der akuten Leukämie nach Chemotherapie) entstehen können, oder andere Tumore, beispielsweise Ovarialtumore oder Lymphome, zu entwickeln.

Für die erfindungsgemäß zu behandelnden präneoplastischen Läsionen kommen daher alle Zellen und Gewebe der äußeren und inneren Organe oder der Oberflächenstrukturen des Säugetierkörpers bzw. des Menschen in Betracht.

Demzufolge liegt der Kern der vorliegenden Erfindung in der systemischen Verwendung eines Arzneimittels zur therapeutischen und/oder prophylaktischen Behandlung von präneoplastischen Läsionen, welches mindestens ein Cytokin oder eine Kombination aus mindestens zwei Cytokinen enthält. Bevorzugt wird das systemisch zu verabreichende Arzneimittel zur Behandlung und/oder Prophylaxe von solchen präneoplastischen Läsionen, die aufgrund physikalischer und/oder chemischer Einwirkungen auf einen Säugetierorganismus hervorgerufen werden können.

Die erfindungsgemäße Verwendung der besagten systemisch zu verabreichenden Cytokine, einzeln oder in Kombination miteinander, auch in Form eines "Kit-of-part", ist daher auch im Sinn eines Adjuvans oder Prophylaktikums zu verstehen, welches vor, bei oder unmittelbar nach einer physikalischen und/oder chemischen Therapie angewendet werden kann, um einem Entstehen präneoplastischer Läsionen und der daraus sich entwickelnden malignen Entartung vorzubeugen, da derartige präneoplastische Läsionen in der Regel klinisch oder morphologisch nicht in Erscheinung treten.

Die erfindungsgemäß zu verwendenden Cytokine, insbesondere IFN und TNF, sind dem Fachmann bekannt und können nach an sich bekannten Methoden, vorteilhafterweise über die Methoden der DNA-Rekombination, hergestellt werden.

Aus immunologischen Gründen ist dem Fachmann bekannt, daß er beim Einsatz von biologisch aktiven, körpereigenen Wirkstoffen vorzugsweise auf speziesspezifische Wirkstoffe zurückgreifen wird. Für die erfindungsgemäße, speziesspezifische Verwendung von Cytokinen wird demnach das aus dem jeweiligen, speziesspezifischen Geweben isolierte Cytokin bevorzugt oder die aus den speziesspezifischen Geweben oder Zellen isolierten Nukleinsäuren (RNA, DNA) zur Herstellung des jeweiligen Cytokins via DNA-Rekombination, insbesondere aber das den jeweiligen, genuinem Cytokin identische Polypeptid mit den bekannten biologischen Aktivitätsspektrum des betreffenden Cytokins. Der Begriff Cytokin ist dem Fachmann geläufig und umfaßt beispielsweise die ihm bekannten Interleukine (z.B. IL-1, IL-2, IL-3, IL-4, IL-5), Interferone (z.B.α, ß, γ), Tumor Nekrose Faktoren (z.B. α, ß), Makrophagen aktivierende Faktoren (MAF), Migrations-Inhibitions-Faktoren (MIF), Wachstums-Faktoren (z.B. Transforming growth factors α,ß). Beispielsweise wird somit das für die erfindungsgemäße Verwendung am Menschen benutzte IFN ein human-IFN, insbesondere human-IFNγ, und der TNF ein human-TNF, insbesondere human-TNFα, sein.

Die Applikationsweise erfolgt erfindungsgemäß über die an sich bekannten systemischen Routen, welche beispielsweise kontinuierlich, z.B. über Mikropumpen, intermittierend oder als Bolus erfolgen kann. Die Dosierung der Cytokine, orientieren sich an den bisher bekannten systemischen Anwendungen, sowie individuell an der Schwere der Erkrankung, an der Ansprechrate und an dem weiteren Verlauf der Erkrankung.

Im allgemeinen kann von einer zu verabreichenden Wirkstoffmenge im Bereich von $10^2$ bis $10^8$ IU, vorzugsweise von $10^4$ bis $10^6$ IU, ausgegangen werden.

Für die Herstellung der jeweiligen Darreichungsformen stehen die dem Fachmann bekannten Hilfs-, Stabilisations- und Trägerstoffe zur Verfügung, wie beispielsweise Albumine, Elektrolyte, Injektions- oder Infusionslösungen.

Legenden zu den Figuren:

Fig 1.: Überlebenskurven von mit 4 x 1.75 Gy bestrahlten C57BL/Ka Mäusen: Kontrolle (—), IFNγ (— - —); TNFα(----); IFNγ plus TNFα(....).

Fig. 2 : Kumulatives Auftreten von Lymphomen bei mit 4 x 1.75 Gy bestrahlten C57BL/Ka Mäusen : Kontrolle (—); IFNγ(— - —); TNFα(----); IFNγ plus TNFα (....).

Das folgende Beispiel soll die Erfindung näher erläutern, ohne den Umfang einzuschränken:

Fünf bis sechs Wochen alte weibliche Mäuse (C57BL/Ka, Stanford University, California) wurden mit 4 Dosen von je 1.75 Gy in wöchentlichen Intervallen ganzkörperbestrahlt (Stabilivolt Siemens, 190 kv, 18 mA, 0.5 mm Cu Filter, Brennpunktdistanz 35 cm, Dosisrate 1.6 Gy/mm).

Mäuse IFNγ, über DNA-Rekombination hergestellt, Fa. Genentech, South San Francisco (California), oder beispielsweise nach Gray P.W., Goeddel, D.V., Proc.Natl. Acad. Sci. USA, 80, 5842-5846, 1983, mit einer spez. Aktivität von $2 \times 10^7$ U/mg und Human TNF-α, über DNA-Rekombination nach bekannten Methoden hergestellt, mit einer spez. Aktivität von $6 \times 10^7$ U/mg, wurden in RPMI 1640 Medium, supplementiert mit 10% fetalem Kalbsserum, verdünnt. Von dieser Verdünnung wurden 200µl Aliquots, enthaltend entweder $4 \times 10^4$ U IFNγ oder 2.5 $\times 10^5$ U TNFα oder ein Gemisch davon intraperitoneal am 1. Tag nach der letzten 1.75 Gy-Bestrahlung injiziert. Drei Injektionen pro Woche (jeden zweiten Tag) innerhalb der ersten 6 Wochen wurden verabreicht.

Jede der drei Gruppen enthielt 16 Mäuse, ebenso enthielt die bestrahlte,untherapierte Gruppe 16 Kontrolltiere.

Die Überlebenskurve wurde nach Kaplan G.L. & Meier, P., J. Am. Statist. Assoc. 53, 457-481, 1958, erstellt. Der Mantel-Haentzel Test wurde zum Vergleich der Unterschiede zwischen den verschiedenen Gruppen verwendet.

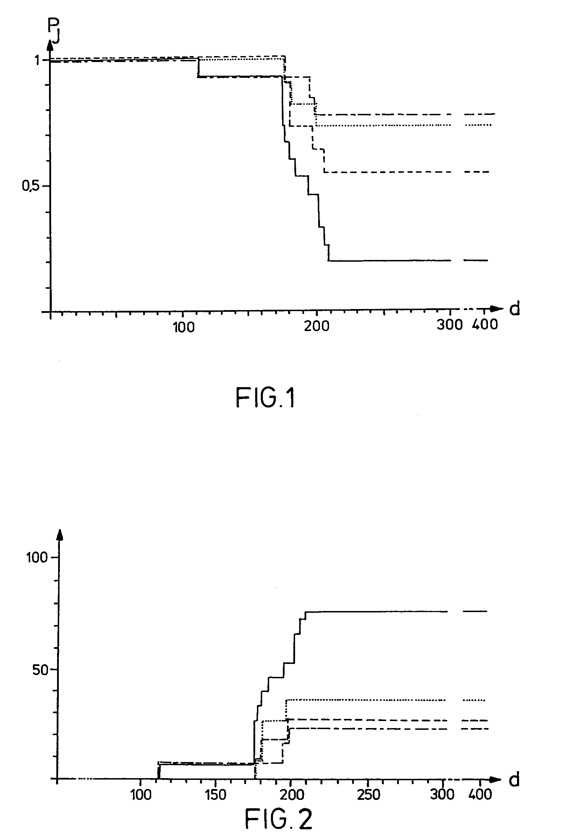

Die Kurvenverläufe der Figuren 1 und 2 zeigen das Überleben und die Entstehung von Lymphomen bei den mit IFNγ und TNFα behandelten, bestrahlten Mäusen im Vergleich zur Kontrollgruppe. In den drei mit Cytokininjektionen behandelten Gruppen war das Aufreten von Thymuslymphomen signifikant niedriger als in den nur bestrahlen, untherapierten Mäusen ($P<0.05$). Ähnliche Ergebnisse wurden für die Überlebensrate erhalten.

## Patentansprüche

1. Verwendung von mindestens einem Cytokin oder einer Kombination aus mindestens zwei Cytokinen zur Herstellung von Arzneimitteln in einer systemisch zu verabreichenden Applikationsform zur Behandlung von präneoplastischen Läsionen.

2. Verwendung von mindestens einem Cytokin oder einer Kombination aus mindestens zwei Cytokinen nach Anspruch 1 zur Behandlung von durch physikalische und/oder chemische Einwirkungen auf einen Säugetierorganismus hervorgerufene präneoplastische Läsionen.

3. Verwendung von mindestens einem Cytokin oder einer Kombination aus mindestens zwei Cytokinen nach Ansprüchen 1 und 2, dadurch gekennzeichnet, daß das Cytokin ein Interferon (IFN) und/oder ein Tumornekrosefaktor (TNF) ist.

4. Verwendung von mindestens einem Cytokin oder einer Kombination von mindestens zwei Cytokinen nach Anspruch 3, dadurch gekennzeichnet, daß das Cytokin IFN-gamma und/oder ein TNF-alpha ist.

5. Verwendung von mindestens einem Cytokin oder einer Kombination von mindestens zwei Cytokinen nach Ansprüchen 1 bis 4, dadurch gekennzeichnet, daß die zu verabreichende systemische Applikationsform $10^2$ bis $10^8$ IU des jeweiligen Cytokins enthält.

6. Verwendung von mindestens einem Cytokin oder einer Kombination von mindestens zwei Cytokinen nach Ansprüchen 1 bis 5, dadurch gekennzeichent, daß die Cytokine aus natürlichen Zellen isoliert oder über gentechnologische Verfahren gewonnen wurden.

## Claims

1. Use of at least one cytokine or a combination of at least two cytokines for preparing pharmaceutical compositions in a form for systemic administration for the treatment of preneoplastic lesions.

2. Use of at least one cytokine or a combination of at least two cytokines according to claim 1 for the treatment of preneoplastic lesions caused by physical and/or chemical effects on a mammalian organism.

3. Use of at least one cytokine or a combination of at least two cytokines according to claims 1 and 2, characterised in that the cytokine is an interferon (IFN) and/or a tumour necrosis factor (TNF).

4. Use of at least one cytokine or a combination of at least two cytokines according to claim 3, characterised in that the cytokine is IFN-gamma and/or a TNF-alpha.

5. Use of at least one cytokine or a combination of at least two cytokines according to claims 1 to 4, characterised in that the preparation for systemic administration contains $10^2$ to $10^8$ IU of the particular cytokine.

6. Use of at least one cytokine or a combination of at least two cytokines according to claims 1 to 5, characterised in that the cytokines are isolated from natural cells or obtained by genetic engineering methods.


**Revendications**

1. Utilisation d'au moins une cytokine ou d'une combinaison d'au moins deux cytokines pour la préparation de médicaments sous une forme d'application à administrer par voie systémique pour le traitement des lésions prénéoplasiques.

2. Utilisation d'au moins une cytokine ou d'une combinaison d'au moins deux cytokines selon la revendication 1 pour le traitement de lésions prénéoplasiques provoquées par des influences physiques et/ou chimiques sur un organisme de mammifère.

3. Utilisation d'au moins une cytokine ou d'une combinaison d'au moins deux cytokines selon les revendications 1 et 2, caractérisée en ce que la cytokine est un interféron (IFN) et/ou un facteur de nécrose des tumeurs (TNF).

4. Utilisation d'au moins une cytokine ou d'une combinaison d'au moins deux cytokines selon la revendication 3, caractérisée en ce que la cytokine est un IFN gamma et/ou un TNF alpha.

5. Utilisation d'au moins une cytokine ou d'une combinaison d'au moins deux cytokines selon les revendications 1 à 4, caractérisée en ce que la forme d'application systémique à administrer contient $10^2$ à $10^8$ UI de cytokine correspondante.

6. Utilisation d'au moins une cytokine ou d'une combinaison d'au moins deux cytokines selon les revendications 1 à 5, caractérisée en ce que les cytokines ont été isolées à partir de cellules naturelles ou ont été obtenues par un procédé de génie génétique.

FIG.1

FIG.2